# EUROPÄISCHE PATENTSCHRIFT

(11) **EP 0 789 584 B1**
(45) Veröffentlichungstag und Bekanntmachung des Hinweises auf die Patenterteilung: **19.09.2001**
(21) Anmeldenummer: 95938419.9
(22) Anmeldetag: 06.11.1995
(51) Int. Cl.: A61K 38/44, A23L 1/015, A61K 9/127

(54) **APPLIKATION VON SOD IN LIPOSOMEN**
APPLICATION OF SUPEROXIDE DISMUTASE IN LIPOSOMES
APPLICATION DE SUPEROXIDE DISMUTASE DANS DES LIPOSOMES

(30) Priorität: 04.11.1994 EP 94117409
(43) Veröffentlichungstag der Anmeldung: 20.08.1997
(73) Patentinhaber: Polymun Scientific Immunbiologische Forschung GmbH, 1190 Wien (AT)
(72) Erfinder: KATINGER, Hermann, A-1190 Wien (AT); VORAUER-UHL, Karola, A-1040 Wien (AT); FÜRNSCHLIEF, Eckhard, A-1050 Wien (AT)
(74) Vertreter: Büchel, Kurt F., Dr.
(86) Internationale Anmeldenummer: EP9504352
(87) Internationale Veröffentlichungsnummer: WO9614083

(56) Entgegenhaltungen:
- EP-A- 0 207 039
- EP-A- 0 457 910
- WO-A-87/01387
- DATABASE WPI Week 8820 Derwent Publications Ltd., London, GB; AN 88-136325 & JP,A,63 077 824 (TOYO SODA MFG KK) , 8.April 1988
- C.R. SEANCES SOC. BIOL. FIL., Bd. 179, Nr. 4, 1985 Seiten 429-439, A.M. MICHELSON ET AL. 'La superoxide dismutase et la pathologie des radicaux libres.'
- DATABASE WPI Week 9320 Derwent Publications Ltd., London, GB; AN 93-164366 & JP,A,05 097 694 (DENKI KAGAKU KOGYO KK) , 20.April 1993
- DATABASE WPI Week 9006 Derwent Publications Ltd., London, GB; AN 90-041606 & JP,A,01 319 427 (MIZUSHIMA) , 25.Dezember 1989
- AM. REV. RESPIR DIS., Bd. 132, Nr. 1, 1985 Seiten 164-167, XP 000566027 R.V. PADMANABHAN ET AL. 'Protection against pulmonary oxygen toxicity in rats by the intratracheal administration of liposome-encapsulated superoxide dismutase or catalase.'
- BULL. CANCER, Bd. 80, Nr. 9, 1993 Seiten 799-807, J.L. LEFAIX ET AL. 'La fibrose cutanéo-musculaire radio-induite: efficacité thérapeutique majeure de la superoxide dismutase Cu/Zn liposomiale.'
- DERMATOLOGICA, Bd. 179, Nr. SP.1, 1989 Seiten 101-106, Y. NIWA 'Lipid peroxides and superoxide dismutase (SOD) induction in skin inflammatory diseases, and treatment with SOD preparations.'
- AM. REV. RESPIR. DIS., Bd. 131, Nr. 4, 1985 Seiten 633-637, XP 000565349 R.J. MCDONALD ET AL 'Effect of superoxide dismutase encapsulated in liposomes or conjugated with polyethylene glycol on neutrophil bactericidal activity in vitro and bacterial clearance in vivo.'
- EUR.J. DERMATOL., Bd. 4, Nr. 5, 1994 Seiten 389-393, XP 000565353 A.A.YOUSSEFI ET AL. 'Oxyradical involvement in puva-induced skin reactions. Protection by local application of SOD.'
- FREE RADICAL BIOLOGY & MEDICINE, Bd. 16, Nr. 6, 1994 Seiten 821-824, XP 000565354 D.B. JACOBY ET AL. 'Influenza virus inducec expression of antioxidant genes in human epithelial cells.'
- DRUGS EXP. CLIN. RES., Bd. 17, Nr. 2, 1991 Seiten 127-131, XP 000565350 Y. MIZUSHIMA ET AL. 'Topical application of superoxide dismutase cream.'

## Beschreibung

Die vorliegende Erfindung bezieht sich auf die Verwendung von Superoxid-Dismutase (SOD), vorzugsweise von rekombinanter humaner SOD in Liposomen, zur Herstellung pharmazeutischer Zubereitungen, gegebenenfalls in einer Mischung mit Hyaluronsäure und/oder mindestens einem physiologisch akzeptablen Träger und/oder gegebenenfalls weiteren Zusätzen, zur therapeutischen und/oder prophylaktischen Anwendung gegen erhöhte Konzentrationen an Superoxidradikalen und/oder dadurch verursachte Schädigungen.

Superoxidradikale sind ausserordentlich reaktive Zwischenformen des natürlichen Sauerstoffmoleküls und können durch diese Eigenschaft organische Verbindungen in den Zellen des menschlichen Körpers irreversibel schädigen. Als Schutz vor der gefährlichen Wirkung dieser Superoxid-Radikale verfügen die Zellen über ein Enzym, welches in der Lage ist, solche Superoxid-Radikale rasch in das schneller metabolisierbare und weniger giftige Wasserstoffperoxid (H₂O₂) umzuwandeln.

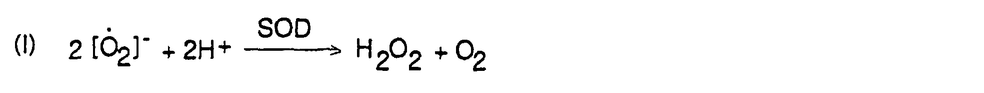

Im Anschluss daran wird normalerweise das noch immer giftige Wasserstoffperoxid durch das Enzym Katalase in die ungefährlichen Bestandteile Wasser und Sauerstoff zerlegt. 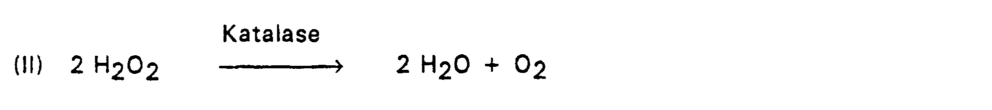 Das Enzym Superoxiddismutase (SOD) kommt sowohl im menschlichen und tierischen Körper als auch in Pflanzen und vermutlich allen Mikroorganismen vor, welche mit Luftsauerstoff direkt in Kontakt kommen (aerobe Bakterien und Pilze). In den Zellen höherer Organismen (Eukaryonten) gibt es hauptsächlich zwei Typen dieser SOD: eine Mangan enthaltende SOD, die in den Mitochondrien lokalisiert ist und der bakteriellen SOD sehr ähnlich ist, und eine zweite, welche im Zytosol frei vorliegt und Kupfer- und Zinkatome enthält.

Im nachfolgenden soll - soweit nicht anders erwähnt - unter dem Begriff SOD hauptsächlich Cu,Zn-SOD, ausgenommen jene aus Rinderblut-Erythrozyten, sowie bakterielle oder mitochondriale Mn-SOD und/oder Fe-SOD, sowie rekombinante humane Cu,Zn-SOD (rhSOD) verstanden werden.

Superoxid-Dismutase ist unter dem Namen Orgotein bereits seit 1939 bekannt, die Dismutase-Aktivität wurde hingegen erst 1969 von McCord und Fridovich entdeckt und beschrieben. Ihre praktische Verwendung war in der Vergangenheit insbesondere durch die Kurzlebigkeit bzw. kurze biologische Verfügbarkeit des Proteins unter natürlichen Umständen begrenzt, was sich natürlich auf die Häufigkeit der Dosierintervalle, die zu wählenden Therapie-Dosen und die damit verbundenen Kosten nachteilig auswirkt.

Die meistuntersuchte und verwendete SOD war bislang die Cu,Zn-SOD aus Rinderblut-Erythrozyten. Nachdem in der klinisch-therapeutischen Anwendung von Rinderblut-SOD, vor allem gegen arthritische Beschwerden, schwere und z.T. sogar tödliche Nebenwirkungen aufgetreten sind, wurden beispielsweise in Östereich Präparate mit Rinderblut-SOD verboten. Einen Ausweg aus dieser Situation bietet vor allem die Herstellung von rekombinanter humaner SOD, wie beispielsweise in AT 397.812 (Polymun Scientific, 1994) beschrieben.

Um die Kurzlebigkeit bzw. kurze biologische Verfügbarkeit des Wirkstoffes SOD besser in den Griff zu bekommen, bietet sich unter anderem die Inkorporation von SOD-Molekülen in Liposomen an (Senga et al. 1990. Transplant.Proc. 22:2025).

Bereits die ersten Einsatzversuche betrafen die Behandlung von Entzündungen und entzündlichen Vorgängen an der Haut. Jedoch wird in der Literatur zwischenzeitlich auch von Einsätzen bei Osteoarthritis und rheumatischer Arthritis berichtet (Hartmann et al. 1986. Proc.Natl.Acad.Sci. U.S.A. 83:7142; Bannister et al. 1987. Critical Rev.Biochem. 22:111). Auch wird - speziell im Bereich der Medizin - berichtet, dass SOD die Lagerfähigkeit von Organen zum Zwecke einer späteren Transplantation (Olson et al. 1988, Transplant.Proc. 20:961) verbessern soll, und auch ein Einsatz zur Nahrungsmittel-Konservierung wird bereits erwähnt (WO 85/01503).

Primäres Ziel der vorliegenden Erfindung ist es, pharmazeutische Zubereitungen zur Verfügung zu stellen um SOD, vorzugsweise rhSOD eingeschlossen in schützenden Liposomen, schonend, effektiv und mit einer besseren Bioverfügbarkeit an die zu behandelnden Stellen des Körpers heranzubringen. Insbesondere ist es den Erfindern gelungen, trotz eines noch immer bestehenden allgemeinen Vorurteils der Fachwelt, liposomal verpackte SOD erfolgreich bei Verbrennungen und Verbrühungen sowie bei Strahlenschädigungen, hervorgerufen beispielsweise durch UV-Strahlen oder ionisierende Strahlen, einzusetzen, insbesondere durch äusserliche Anwendung. Im Falle der Strahlenexposition ist neben dem therapeutischen Einsatz auch eine prophylaktische Anwendung, beispielsweise zusammen mit einem strahlenfilternden oder strahlenabsorbierenden Schutzmittel, möglich.

Ein weiteres Ziel ist es, eine pharmazeutische Zubereitung auf Basis SOD in Liposomen zur Verfügung zu stellen, die die genannten Nachteile des Standes der Technik überwindet und damit zusätzliche Anwendungsgebiete, insbesondere im Bereich der Kosmetik, eröffnet.

Ebenso ist es ein Ziel der vorliegenden Erfindung, SOD, vorzugsweise rhSOD eingeschlossen in schützenden Liposomen, schonend, effektiv und mit einer besseren Haltbarkeit sowie länger anhaltender Wirkung an die zu behandelnden organischen Materialien, beispielsweise pflanzliche oder tierische Gewebe, Organe, Organ- oder Gewebetransplantate, kosmetische Präparate auf organischer Basis und/oder Lebensmittel heranzubringen.

Ein besonderes Ziel der vorliegenden Erfindung ist die Nutzung des synergistischen Effektes einer Mischung von Hyaluronsäure und SOD. Hyaluronsäure ist in der Fachwelt seit kurzem auch für ihre "Radikalfänger-Eigenschaften" bekannt, und ihr Einsatz in der Wundbehandlung ist wiederholt beschrieben worden (Amgen, WO 9214480, 1992). Auch die Verwendung von Hyaluronsäure zusammen mit Colony Stimulating Factor (CSF) oder Platelet Derived Growth Factor (PDGF) zur Beschleunigung der Wundheilung wurde in der Literatur beschrieben (Zymogenetics, US 5128321, 1992), ebenso wie Hyaluronsäure als Zusatz in Kosmetika und pharmazeutischen Präparaten (Shiseido, WO 9104279, 1991).
Bislang nicht beschrieben ist hingegen - erstaunlicherweise - eine Kombination von Hyaluronsäure und SOD, insbesondere eine Mischung von Hyaluronsäure und liposomal inkorporierter SOD. Inwieweit hier ein Vorureil der Fachwelt besteht oder bestanden hat, kann zum gegenwärtigen Zeitpunkt nicht hinreichend beurteilt werden. Jedenfalls hat sich im Zuge der Experimente, die schliesslich zur vorliegenden Erfindung geführt haben, überraschend herausgestellt, dass Hyaluronsäure in fast idealer Weise die Wirkung von SOD, auch von liposomal inkorporierter SOD, unterstützt und verstärkt.

Verbrennungen und Verbrühungen und/oder eine gesundheitsschädigende Dosis an ionisierender oder UV-Strahlung induzieren unter anderem eine kaskadenähnlich ablaufende Reaktion, die beispielsweise für das "Nachbrennen" (=Zunahme der nekrotischen Schädigung) in der 2. und vor allem 3. Dimension verantwortlich ist. Das Problem in der herkömmlichen Sofortbehandlung von Brandwunden und Strahlenschäden besteht in der Praxis darin, dass es bisher ausser der Kaltwasser-Soforttherapie kein lokal anwendbares Mittel gibt, das man als Soforttherapeutikum zur Unterdrückung von Nachbrenn-Phänomenen einsetzen könnte. Von fetthaltigen oder fettfreien Brandsalben oder -gelen ist in dieser Richtung kein vorteilhafter, heilender Effekt bekannt oder zur Zeit zu erwarten.
Es ist daher mehr als erstaunlich, dass SOD in Liposomen bislang zur lokalen, äusserlichen Sofort-Wundbehandlung - ausser in Form von Injektionen direkt in die Wunde - weder in der Tiermedizin noch in der Humanmedizin eingesetzt wird, zumal SOD in Liposomen bereits seit mehr als 5 Jahren bekannt ist (JO3101626, 1989).

Es wird in der Literatur allerdings auch beschrieben, dass die Verwendung von SOD zur Unterstützung der Wundheilung bzw. des Überlebens von Balb/c Mäusen mit Verbrennungen und künstlich erzeugter Infektion, keinen signifikanten Heilerfolg erzielen konnte (Fang et al., The Journal of Trauma, Vol.30. No.4 :453-456, 1990).

Trotz dieser Nachteile des Standes der Technik und dem möglichen Vorurteil der Fachwelt gegenüber liposomal dargebotenen Wirkstoffen zur topischen, insbesondere äusserlichen, Anwendung am Körper bzw. an der Körperoberfläche, ist es den Erfindern dank ihren erfinderischen Annahmen in unerwarteter Weise gelungen, SOD in Liposomen erfolgreich gegen thermische und strahleninduzierte Haut- und Gewebeschädigungen einzusetzen.

Folgende Überlegungen spielten dabei eine Rolle: durch das Trauma selbst, auch im Falle des intensiven Sonnenbrandes, fällt die Barriere der Hornschicht mehrheitlich oder sogar völlig weg, d.h. vor der Entwicklung einer Ödemformation bestünde die Chance zur optimalen Wirkstoffentfaltung in Richtung Lederhaut und subcutan unter das Corium, insbesondere im Falle einer frühzeitigen Applikation von SOD, vorzugsweise rhSOD, in Liposomen.

Der überraschende Erfolg bei der äusserlichen topischen Behandlung der erwähnten Gewebeschädigungen ist vermutlich weiters darauf zurückzuführen, dass durch die Gewebeverletzung verstärkt Makrophagen gebildet werden, welche im Zuge ihrer immunologischen Schutzfunktion (Infektionsabwehr, Beseitigung von Zellbruchstücken) mit den Liposomen in Kontakt treten, deren Lipidschicht auflösen und dabei den Inhalt - die SOD-Moleküle - freisetzen, worauf diese wiederum ihre Superoxid abbauende und damit auch gewebeschützende Aktivität entfalten können.

Im Zuge der Experimente, die schliesslich zur vorliegenden Erfindung geführt haben, konnte ausserdem überraschend herausgefunden werden, dass dank des Einschlusses der SOD-Moleküle in Liposomen nicht nur deren Haltbarkeit und Bioverfügbarkeit verlängert, sondern auch die zur Erreichung des gewünschten Effektes nötige Konzentration an SOD - gegenüber SOD-Zubereitungen ohne Liposomen - um bis zu einem Faktor 10 reduziert werden konnte, ohne dabei Wirkungsverluste in Kauf nehmen zu müssen. Darüber hinaus wird durch die liposomale Darreichungsform auch eine physiologisch günstigere Dosierung des Wirkstoffes am Ort des Bedarfes erreicht, was sich natürlich auf die Menge und/oder Häufigkeit der Dosierungen und die damit verbundenen Kosten sehr vorteilhaft auswirkt. Dieser vorteilhafte Effekt der physiologisch günstigeren Dosierung liposomal inkorporierter SOD vor Ort kommt auch bei Entzündungen und entzündlichen Prozessen an der Körperoberfläche und im Inneren des Körpers, sowie bei Verfahren zur Verbesserung der Haltbarkeit organischer Materialien zum Tragen.

Es gibt allerdings auch Vermutungen, dass möglicherweise nicht nur die direkte enzymatische Wirkung des SOD-Proteins für den Heilerfolg ausschlaggebend ist. Es ist von anderen Enzymen, z.B. Cytochrom A, Histon, Lysozym, Ribonuclease, bekannt, dass sie beispielsweise in dimerisierter Form zusätzliche Eigenschaften aufweisen, die jene des Monomers beträchtlich erweitern, wobei auf diese Weise das Wirkungsspektrum und der jeweilige Einsatzbereich der dimeren Proteine gegenüber den Monomeren in vorteilhafter Weise ausgedehnt wird (Bartholeyns and Zenebergh, Europ.J.Cancer, Vol.15, 1979, 85-91).

Bei Verwendung von Hyaluronsäure in einer Mischung mit SOD, insbesondere rhSOD, mit oder ohne Liposomen, hat sich überraschenderweise herausgestellt, dass sich ein synergistischer Effekt insofeme ergibt, als sich, insbesondere bei äusserlicher Anwendung, ein - im Vergleich zu einer SOD-behandelten Brandwunde ohne Hyaluronsäure - wesentlich glatteres und elastischeres Gewebe neu bildet. Dieser Effekt konnte auch im Falle von entzündeten Wunden an der Körperoberfläche festgestellt werden. Hyaluronsäure dient dabei im Gemisch mit liposomaler SOD sowohl als synergistisch wirkender Radikalfänger als auch als pharmazeutisch akzeptabler Trägerstoff.

Im Rahmen der Experimente, welche der vorliegenden Erfindung zugrunde liegen, hat sich ausserdem unerwarteterweise herausgestellt, dass sich bei Verwendung einer Zubereitung von Hyaluronsäure in einer Mischung mit SOD, insbesondere rhSOD in Liposomen, ein synergistischer Effekt insoferne ergibt, als eine im Vergleich zur SOD-Behandlung ohne Hyaluronsäure signifikant längere Haltbarkeit bzw. Wirkungsdauer der Zubereitung selbst und damit auch eine längere Haltbarkeit der behandelten Materialien resultiert; dieser Effekt zeigt sich insbesondere dann, wenn die SOD frei in der Zubereitung, d.h. nicht in Liposomen eingeschlossen, vorliegt. Sowohl SOD allein als auch Hyaluronsäure allein waren in dieser Hinsicht einer Mischung beider Komponenten deutlich unterlegen.

Dieser synergistische Effekt einer Mischung von SOD und Hyaluronsäure wird möglicherweise dadurch begünstigt, dass die Hyaluronsäure sowohl die Phospholipdschichten der Liposomen und/oder die empfindlichen SOD-Moleküle vor schädigenden, d.h. vornehmlich oxidierenden, Einflüssen von aussen schützt, als auch nach Inaktivierung der SOD noch einen eigenen, wenn auch nur geringen, Beitrag zur Superoxidradikal-Beseitigung leistet.

Die vorliegende Erfindung enthält mehrere verschiedene Ausführungsformen der Anwendung von SOD, insbesondere rhSOD in Liposomen, mit oder ohne Hyaluronsäure und gegebenenfalls in Kombination mit Trägerstoffen und/oder weiteren Zusätzen, für die Herstellung von pharmazeutischen Zubereitungen gegen eine Reihe von Indikationen.

Eine Ausführungsform bzw. ein Merkmal der Erfindung bezieht sich auf die Verwendung von SOD, insbesondere von rekombinanter humaner SOD (rhSOD) in Liposomen, gegebenenfalls in einer Mischung mit Hyaluronsäure und/oder einem physiologisch akzeptablen Träger sowie gegebenenfalls weiteren, für galenische Formulierungen üblichen Zusätzen, zur Herstellung von pharmazeutischen Zubereitungen gegen Verbrennungen, Verbrühungen und Strahlenschädigungen, insbesondere solchen, die durch UV-Strahlen verursacht werden.

In einer erfindungsgemässen Ausführungsform wird z.B. in Liposomen inkorporierte rhSOD als keimfreies Wundgel formuliert, wobei der gegebenenfalls beigemischte Träger fettarm oder fettfrei ist und aus der Gruppe der organischen und anorganischen Hydrogele stammt, und direkt auf die Brandwunde aufgetragen. Neben einer klinischen Anwendung für Patienten mit zweit- und drittgradigen Verbrennungen, z.B. aus Unfallsituationen, kommt eine solche Formulierung insbesondere auch Personen zugute, welche sich beim Sonnenbaden am Wasser oder im Schnee grossflächige oder lokal begrenzte Sonnenbrände zugezogen haben.

Gerade bei Brandwunden hat es sich auch als besonders vorteilhaft erwiesen, wenn man die SOD-haltigen Liposomen, in flüssiger Form durch Aufsprühen aus einer Spraydose oder Sprühflasche auf die verletzte Stelle aufbringt. Man vermeidet dadurch den direkten Kontakt der Wunde mit den Fingern oder einem sonstigen Hilfsmittel zur Auftragung, beispielsweise eines Gels, und reduziert damit die Gefahr einer zusätzlichen Infektion.

In einer anderen Ausführungsform wird liposomal inkorporierte SOD, gegebenenfalls mit Hyaluronsäure und/oder weiteren Zusätzen, auf-vorzugsweise - sterile Wundpflaster und/oder Wundabdeckungen aufgebracht, um auf diese Weise ein rasch verfügbares und leicht handhabbares Mittel zur effektiven Behandlung und/oder Selbst-Behandlung, von kleineren und mittelgrossen Brandwunden bzw. Hautverbrennungen bereits am Ort des Geschehens an der Hand zu haben. Der besondere Vorzug dieser Anwendungsform besteht in der einfachen Art und Weise der Anwendung, wie sie auch von medizinisch ungeschulten Personen sicher vorgenommen werden kann, beispielsweise von Eltern, deren Kind sich am Herd die Finger verbrannt oder mit heissem Wasser verbrüht hat.

Ein besonderer Aspekt der vorliegenden Erfindung ist die Verwendung von SOD, insbesondere rhSOD in Liposomen, zur Herstellung von Zubereitungen, die vor, während oder nach einer Strahlenexposition eingesetzt werden. Für rein therapeutische Anwendungen, d.h. nach bereits erfolgter übermässiger Strahlenbelastung, beispielsweise bei einem Sonnenbrand, empfiehlt sich zur Milderung der Folgen der Strahleneinwirkung der Einsatz der erfindungsgemässen SOD-Zubereitungen, vorzugsweise rhSOD in Liposomen, gegebenenfalls in einer Mischung mit mindestens einem physiologisch akzeptablen Träger und/oder Hyaluronsäure, sowie gegebenenfalls weiteren Zusätzen, in Form von Emulsionen, Suspensionen, Lösungen, Lotions oder allenfalls niedrigviskosen Salben oder Gelen.

Besonders vorteilhaft sind die Zubereitungen, welche mittels einer Sprühvorrichtung, beispielsweise einem Spray, auf die geschädigten Hautpartien aufgebracht werden können. Einerseits wird dadurch der direkte Kontakt und damit eine mögliche Infektion der verletzten Haut mit möglicherweise unreinen Fingern oder sonstigen Hilfsmitteln zur Auftragung der Zubereitung vermieden und andererseits erspart man sich dadurch eine mehr oder weniger schmerzhafte Auftragung durch Verreiben beispielsweise eines höherviskosen Gels.

Zur prophylaktischen und gleichzeitg therapeutischen Anwendung vor und während einer Strahlenexposition eignen sich vor allem jene erfindungsgemässen Zubereitungen, welche neben SOD und gegebenenfalls Hyaluronsäure auch noch mindestens ein strahlenfilterndes oder strahlenabsorbierendes Schutzmittel, vorzugsweise einen Lichtfilter oder UV-Absorber, insbesondere einen UVB-Filter, enthalten. Zusätzlich können auch noch weitere Substanzen, vor allem Hautpflegefaktoren, vorhanden sein.

Durch die Anwesenheit von SOD und gegebenfalls Hyaluronsäure neben üblichen Lichtfilter-Substanzen können die unangenehmen Folgen eines Sonnenbrandes erheblich vermindert werden. Derartige prophylaktisch und therapeutisch wirkende Sonnenschutzpräparate können vorteilhaft bei jeder Art des Sonnenbadens, ob am Strand, im Gebirge, auf See, auf der Schipiste oder in einem Solarium, eingesetzt werden. Insbesondere Menschen mit empfindlicher Haut und jene Hauttypen, bei denen eine Hautbräunung durch UV-Strahlen nicht erfolgt, profitieren am meisten von dieser Anwendungsform der vorliegenden Erfindung.

Eine andere Ausführungsform bezieht sich auf die Herstellung von Zubereitungen zur therapeutischen und/oder prophylaktischen Anwendung bei akuten und chronischen Entzündungen und entzündlichen Prozessen, bei rheumatischen Erkrankungen, insbesondere bei rheumatischen Gelenksentzündungen und/oder Osteoarthritis, aber auch bei Entzündungen der Atemwege, insbesondere bei Bronchitis, Schocklunge (Acute Respiratory Distress Syndrome, ARDS), Emphysemen, und anderen Entzündungsvorgängen, erfolgreich anwendbar. Speziell für den Bereich kosmetischer Anwendungen zur prophylaktischen und/oder therapeutischen Behandlung von lokalen Entzündungen der Haut wie beispielsweise Furunkeln oder Akne, die bekanntermassen für viele Menschen eine erhebliche kosmetische Beeinträchtigung darstellen, eröffnen sich dadurch vielversprechende Möglichkeiten.

In einer erfindungsgemässen Ausführungsform wird z.B. in Liposomen inkorporierte rhSOD als steriles Wundgel formuliert, wobei der gegebenenfalls beigemischte Träger fettarm oder fettfrei ist und aus der Gruppe der organischen und anorganischen Hydrogele stammt, und direkt auf die entzündete Stelle aufgetragen. Ein fettarmer oder fettfreier Träger ist vor allem bei offenen, entzündeten Wunden von Vorteil, da überhöhte Lipidkonzentrationen im Wundbereich zur Entstehung von toxischen Abbauprodukten führen können. Hyaluronsäure ist in solchen Zubereitungen besonders vorteilhaft einsetzbar, entweder als alleiniger Träger oder in einer Mischung mit mindestens einem weiteren Träger der obigen Kategorie. Ausserdem können weitere geeignete Zusätze, wie sie beispielsweise für galenische Formulierungen üblich sind, ebenfalls in den erfindungsgemässen Zubereitungen enthalten sein.

Gerade bei oberflächlichen Entzündungen hat es sich auch als besonders vorteilhaft erwiesen, wenn man die SOD-haltigen Liposomen in flüssiger Form durch Aufsprühen aus einer Spraydose, Sprühflasche oder sonstigen Sprühvorrichtung auf die enzündete Stelle aufbringt. Man vermeidet dadurch den direkten Kontakt der Wunde mit den Fingern oder einem sonstigen Hilfsmittel zur Auftragung der Zubereitung und reduziert damit die Gefahr einer zusätzlichen Infektion. In bestimmten Fällen kann es aber auch von Vorteil sein, wenn mindestens eine der erfindungsgemässen Zubereitungen auf-gegebenenfalls sterile - Wundabdeckungen, beispielsweise Wundauflagen, Heftpflaster und dergleichen, aufgebracht wird. Solcherart präparierte Wundabdeckungen eignen sich gut für die einfache und rasche Behandlung, gegebenenfalls auch Selbstbehandlung, beispielsweise bei entzündeten Wunden und/oder rheumatischen Gelenksentzündungen.

In einer weiteren Ausführungsform der vorliegenden Erfindung wird liposomal inkorporierte SOD, insbesondere rhSOD, zusammen mit Hyaluronsäure und gegebenenfalls mindestens einem zusätzlichen fettarmen oder fettfreien, Trägerstoff, insbesondere aus der Gruppe der organischen und anorganischen Hydrogele, und gegebenenfalls weiteren Zusätzen, als Injektionslösung formuliert und bei rheumatologischen und/oder orthopädischen Indikationen, beispielsweise rheumatischen Gelenksentzündungen oder Osteoarthritis, erfolgreich angewendet.
Dabei kann die Injektion sowohl direkt in die betroffenen Gelenke (intraartikulär) oder Körperregionen oder auf sonstige parenterale, vorzugsweise intravenöse, Weise erfolgen.

In gewissem Umfang lassen sich Linderungen rheumatisch-arthritischer Leiden auch durch äusserliche Behandlung, beispielsweise durch eine ebenfalls erfindungsgemässe, äusserliche Applikation von SOD in Liposomen, insbesondere im Gemisch mit Hyaluronsäure, erzielen. Dabei können die Wirkstoffe sowohl in Form von Salben oder Gelen, allenfalls in Mischung mit zusätzlichen, vorzugsweise fettarmen oder fettfreien, Trägerstoffen, insbesondere aus der Gruppe der organischen und anorganischen Hydrogele, als auch in Form von Sprays oder Tinkturen formuliert und angewendet werden. Geeignete Zusätze, wie sie für galenische Formulierungen von Pharmazeutika üblich sind, können ebenfalls in den Zubereitungen enthalten sein. In manchen Fällen kann auch eine begleitende, orale Verabreichung von liposomaler SOD in Form von Tabletten, Kapseln, Dragees, Pulvern etc. unterstützend wirken.

In einer weiteren Ausführungsform konnten bestimmte degenerative Erscheinungen wie Emphyseme, insbesondere Hautemphyseme, durch die erfindungsgemässe Anwendung von SOD in Liposomen, gegebenenfalls in einer Mischung mit Hyaluronsäure und/oder mindestens einem physiologisch akzeptablen Träger, erfolgreich abgeschwächt und eingedämmt werden. Möglicherweise spielen auch hier neben der rein enzymatischen Wirkung der SOD noch zusätzliche, phagocytosestimulierende Effekte der SOD-Aggregate in den Liposomen eine synergistische Rolle.

Ähnliches dürfte auch für die erfolgreiche therapeutische Anwendung der pharmazeutischen Zubereitungen der vorliegenden Erfindung bei Entzündungen der Atemwege und der Lunge, wie z.B. Bronchitis, Schocklunge (ARDS) und Lungenemphysem, zum Tragen kommen. In diesem Falle hat sich speziell die Applikation einer Lösung oder Emulsion von liposomal inkorporierter SOD per Inhalation als besonders gut geeignet erwiesen.

Die Anwendung der liposomalen SOD-Zubereitungen ist insbesondere im Bereich der kosmetischen Störungen sehr erfolgreich. Kleine lokale Entzündungen, Furunkel, Akne und ähnliche Erscheinungen sind durch die erfindungsgemässe Anwendung der SOD-haltigen Zubereitungen, insbesondere in Form von Salben, Gelen, Crémes oder in flüssiger Form durch Aufsprühen, wirksam behandelbar. Bei wiederholter, vorzugsweise regelmässiger, Applikation ist auch ein gewisser prophylaktischer Effekt in Bezug auf die Entstehung neuer Hautenzündungen, beispielsweise von Furunkeln, erzielbar. Vielleicht hängt auch dies mit einer durch die SOD-Aggregate möglicherweise ausgelösten oder stimulierten Aktivierung von Phagocytose-Vorgängen zusammen.

Bei Verwendung einer Mischung von liposomaler SOD und Hyaluronsäure erfolgt ausserdem die Abheilung der Entzündung(en) unter Neubildung eines elastischeren und vor allem glatteren Gewebes im Vergleich zu Hyaluronsäurefreien Zubereitungen. Speziell für kosmetische Anwendungen an sichtbaren Körperstellen, also beispielsweise im Gesicht, ist dies ein erheblicher Vorteil.

Die erfindungsgemässen Zubereitungen der vorliegenden Erfindung sind im allgemeinen dann am wirksamsten, wenn die SOD, je nach Anwendungsfall und Art der Applikation, in einer Konzentration von ≥ 0.01 Gew.%, insbesondere von 0.01 bis 5 Gew.% und die gegebenenfalls zusätzlich vorhandene Hyaluronsäure in einem Anteil von ≥ 0.05 Gew.%, insbesondere 0.1 bis 5 Gew.% der fertigen, anwendungsbereiten Zubereitung vorliegt. Für topische Anwendungen hat sich eine Menge von 0.01 bis 1 mg SOD/cm² Läsionsfläche bzw. Körperoberfläche als am besten geeignet erwiesen. Orale oder parenterale Applikationen werden hingegen vorteilhaft mit SOD-Dosen von 0.5 bis 50 mg/kg Körpergewicht durchgeführt, wobei für wiederholte SOD-Gaben im Rahmen einer Therapie die Dosis vorteilhafterweise bei 0.5 bis 10 mg/kg*Tag eingestellt wird.

Die vorliegende Erfindung bezieht sich weiters auf die Anwendung von SOD zur Herstellung von Zubereitungen, die zur Verbesserung der Haltbarkeit von verschiedenen organischen, vorzugsweise biogenen, Materialien eingesetzt werden können, sowie auf Verfahren zur Verbesserung der Haltbarkeit solcher organischer Materialien unter Verwendung der erfindungsgemässen Zubereitungen.

Grundsätzlich kann man gemäss der vorliegenden Erfindung SOD, vorzugsweise rhSOD in Liposomen, in Form einer wässerigen Lösung bzw. Emulsion mit und ohne Hyaluronsäure zubereiten und direkt zur konservierenden Anwendung einsetzen. In verschiedenen Fällen kann jedoch die Beimischung einer oder mehrerer geeigneter Trägersubstanzen und/oder weiterer, für galenische Zubereitungen üblicher, Zusätze von Vorteil sein, um beispielsweise eine bestimmte, gewünschte Applikationsform zu ermöglichen oder zu vereinfachen.
Wesentlich für den erfindungsgemässen Einsatz der Zubereitungen, beispielsweise im Rahmen eines Verfahrens zur Verbesserung der Haltbarkeit der organischer Materialien gemäss der vorliegenden Erfindung, ist jedoch, dass das organische Material zumindest teilweise mit wenigstens einer der hierin beschriebenen Zubereitungen in Kontakt gebracht wird.

In einer Ausführung der vorliegenden Erfindung wird liposomal inkorporierte SOD, insbesondere rhSOD, in einer geeigneten Pufferlösung vorgelegt, um damit Organe oder organische Gewebe, die zum Zwecke einer Transplantation einem Spender entnommen wurden, in dem Zeitraum zwischen Entnahme und Wiedereinpflanzung konservierend zu behandeln. Dabei hat sich eine Zubereitung mit einem SOD-Gehalt von 0,1-1 Gew.% als sinnvoll erwiesen. Die Organ- bzw. Gewebe-konservierende Behandlung erfolgt dabei am besten zuerst durch eine Spülung des betreffenden Organs nach üblichen Methoden der Organkonservierung, insbesondere durch Injektionen oder Infusion der flüssigen Zubereitung in das Organ, beispielsweise ein Herz, eine Niere, eine Leber etc., und vorzugsweise anschliessend durch Eintauchen oder noch besser Einlegen des jeweiligen Organs oder Gewebeteiles in ein Bad, welches ebenfalls liposomale SOD entweder in dem gleichen oder einem anderen geeigneten Puffer enthält. Auf diese Weise gelingt es, einerseits den Zeitraum der Zwischenlagerung beträchtlich zu verlängern und andererseits mögliche Organ- bzw. Gewebeschädigungen nach der Reimplantation zu vermindern.

Erfreulicherweise hat sich gezeigt, dass in diesem Fall durch Beimischung von Hyaluronsäure, beispielsweise in Konzentrationen von ca. 0.05 - 2.5 Gew.%, zur bestehenden Emulsion aus liposomaler SOD und Puffer ein zusätzlich synergistischer Effekt der oben beschriebenen Art erzielt werden konnte.

In einer anderen Ausführungsform wird liposomale SOD in einer flüssigen Zubereitung - mit und ohne Hyaluronsäure - auf Lebensmitteln augebracht, beispielsweise mittels Sprühflaschen, Spraydosen oder sonstigen Sprühvorrichtungen. Insbesondere leichtverderbliche Fleischwaren, aber auch Molkereiprodukte aller Art sowie Obst und Gemüse konnten durch diese einfache und leicht zu handhabende Massnahme in ihrer Haltbarkeit entscheidend verbessert werden. Die liposomal verpackte SOD fungiert in diesem Falle quasi als Antioxidans.

Auch Tauchbäder, welche wenigstens eine der erfindungsgemässen Zubereitungen enthalten und in welche die organischen Materialien, beispielsweise verschiedene Lebensmittel, über einen gewünschten Zeitraum eingetaucht oder eingelegt werden, sind geeignet, um die Wirkstoffe SOD und gegebenenfalls Hyaluronsäure mit dem organischen Material in Kontakt zu bringen.

Eine weitere Ausführungsform der vorliegenden Erfindung bezieht sich auf den Einsatz von freier SOD, insbesondere rhSOD, oder liposomaler SOD, in einer Mischung mit Hyaluronsäure und gegebenenfalls mindestens einem weiteren geeigneten Träger zur Verbesserung der Haltbarkeit von kosmetischen Präparaten auf organischer Basis, insbesondere von Hautpflegemitteln. Versuche haben gezeigt, dass sowohl SOD in Liposomen allein als auch freie oder liposomal inkorporierte SOD in einer Mischung mit Hyaluronsäure, hervorragend geeignet ist, als Zusatz zu dem organischen Material in kosmetischen Präparaten wie Salben, Crémes, Gelen, Ölen, Lotions, Wässern, Milks und dgl. deren Haltbarkeit zu verbessern. Insbesondere im Falle der Mischung mit Hyaluronsäure ergibt sich noch ein zusätzlich positiver Effekt in Hinblick auf eine gewisse Erhöhung der Geschmeidigkeit und Glättung der Haut nach Anwendung solcherart verbesserter Kosmetika.

Die Konzentration an SOD liegt in diesem Anwendungsfall bevorzugt zwischen 0.1 und 5 Gew.%, der Anteil an gegebenenfalls vorhandener Hyaluronsäure wird hingegen am besten mit 0.5-5 Gew.%, bezogen auf die endfertige Zubereitung, gewählt. Falls ausserdem ein oder mehrere Trägersubstanzen dazugemischt werden sollen, dann sollen diese vorzugsweise fettarm oder fettfrei sein und gegebenenfalls aus der Gruppe der organischen und anorganischen Hydrogele stammen.

Zum Zwecke der Verbesserung der Haltbarkeit von organischen Materialien wie beispielsweise pflanzlichen und tierischen Geweben, Organtransplantaten, Lebensmitteln, insbesondere leichtverderblichen Fleisch- und Wurstwaren, oder Kosmetika auf organischer Basis, haben sich SOD-Konzentrationen von 0.1 bis 100 mg/kg organisches Material als sinnvoll erwiesen.

Um die erfindungsgemässen Einsatzmöglichkeiten und die Wirkungsweise der hierin beschriebenen Zubereitungen noch weiter zu erläutern, werden im folgenden einige Beispiele angeführt. Die Beispiele dienen dem besseren Verständnis und sollen in keiner Weise den Inhalt oder Umfang der vorliegenden Erfindung einschränken.

### Beispiel 1: Anwendung bei Verbrühungen

70 Albinokaninchen wurden in 7 Gruppen (A - G) eingeteilt. Jedem Kaninchen wurde eine Fläche von 5 x 10 cm auf dem Rücken abrasiert. Nach tiefer Narkotisierung wurden die Kaninchen an der abrasierten Rückenfläche einzeln in eine Wanne mit einer Öffnung von genau 3 x 5 cm, gefüllt mit heissem Wasser von 93,5 - 95°C, für exakt 16 Sekunden lang eingetaucht. Dadurch entstanden Verbrühungen zweiten bis dritten Grades. Die Verbrühungsflächen wurden gemäss untenstehender Tabelle 1 behandelt und mit sterilem Wundverband mit Aluminiumeinlage sorgfältig bedeckt. Der Verband wurde täglich gewechselt.

Die Liposomen mit inkorporierter rhSOD wurden folgendermassen hergestellt: Als Präparationsmethode für die grossteils unilamellaren Liposomen wurde die Einspritzmethode nach Batzri und Korn (Batzri, S. Korn, E.D. 1973, Biochim.Biophys. Acta 298:1015-19) verwendet. Die Lipidbestandteile Dipolmitoyl-sn-glycerophosphocholin (DPPC), cholesterin und Stearylamin werden in einem molaren Verhältnis von 7:1:2 in 96% Ethanol gelöst. Die Lipidkonzentration der Liposomenlösung beträgt 10 µmol/ml. Das Ethanolvolumen wird so gewählt, dass die Ethanolkonzentration in der Präparation unter 7% liegt. Die lyophilisierte rhSOD wird in PBS gelöst. Die Proteinkonzentration der wässrigen Phase beträgt 10 mg/ml. Beide Lösungen werden auf 37°C temperiert. Die Liposomen werden durch kontinuierliche Injektion der ethanolischen Phase in die wässrige Phase produziert. Zum Abtrennen der nicht inkorporierten rhSOD bzw. des Ethanols wird die Liposomenlösung gegen PBS diafiltriert (Ultra/Diafiltrationseinheit Fa. Amicon; Membran: YM 100).
Für die Endformulierung wird das Carbogel (Carbopol® , stark saure Acrylsäure-Polymerisate mit hohem Molekulargewicht, DAB9) in aqua dest. rehydriert und der pH-Wert auf 7.5 eingestellt. Die Liposomenlösung und das rehydrierte Gel werden homogen vermischt und bei 4°C gelagert. Der gesamte Herstellungsprozess wird mit sterilen Lösungen und im Laminarflow (sterile Reinraumbank) durchgeführt.

Herstellung des liposomal inkorporierten rhSOD-Hyaluronsäure-Gels: Lyophilisierte Hyaluronsäure wird in der wässrigen Liposomenlösung rehydriert und das fertige Gel bei 4°C gelagert.

Die oben erwähnten Versuchstiere wurden laufend von einem Tierarzt untersucht, es wurden keine Anzeichen einer klinischen Erkrankung der Tiere während des Versuchsablaufes festgestellt. Die Tiere zeigten auch keine posttraumatischen Schmerzerscheinungen nach dem Erwachen aus der Narkose, trotzdem wurde vorsichtshalber eine posttraumatische Analgesie eingeleitet.

Die Gruppe F blieb als Kontrollgruppe unbehandelt. Die übrigen Gruppen wurden nach folgendem Schema behandelt:

**Tabelle 1**

| **Test- gruppe** | **Anzahl der Tiere** | **Identifizierungs- nummern** | **Dosis und Verabreichung** |
|---|---|---|---|
| A | 10 | 1 - 10 | 0.1 mg rhSOD / cm² der Läsion, in Liposomen; |
| | | | 2x am ersten Tag appliziert |
| B | 10 | 11 - 20 | 1 mg rhSOD / cm² der Läsion, in Gel ohne Liposomen; 1x am ersten Tag appliziert |
| C | 10 | 21 - 30 | 1 mg rhSOD / cm² der Läsion; |
| | | | intraläsional injiziert |
| D | 10 | 31 - 40 | Kontrollgruppe: Gel mit leeren Liposomen; |
| | | | 1x am ersten Tag appliziert |
| E | 10 | 41 - 50 | Kontrollgruppe: Gel ohne Liposomen; |
| | | | 1x appliziert am ersten Tag |
| F | 10 | 51 - 60 | Kontrollgruppe: ohne Behandlung |
| G | 10 | 61-70 | 0.1 mg rhSOD in Liposomen/ cm² der Läsion, mit Hyaluronsäure; 2x am ersten Tag appliziert |

Statistisch ausgewertet wurden die Grösse der Wunden (Planimetrie, fotografische Dokumentation), der Lokalstatus (Farbe, Vorliegen von Nekrosen, Epithelisierungszeichen, Kontrakturen, Behaarung, Kutimetrie, Granulationsgewebe), histopathologische Hautuntersuchungen und makroskopisch der allgemeine Heilungsverlauf (Photodokumentation).

Für die untersuchten Parameter konnte ein positiver Einfluss der in Liposomen inkorporierten rhSOD auf die Heilungs- und Regenerationsprozesse bei den behandelten Versuchstieren festgestellt werden. Am deutlichsten war die Wirkung der rhSOD 24 Stunden nach der Läsionssetzung. So gab es beispielsweise zwischen den Testgruppen einen signifikanten Unterschied in der Läsionsgrösse, wobei bei den rhSOD-behandelten Tieren die Übergangszone der Nekrose in die umliegende nicht verbrühte Haut deutlich weniger von der Verbrühung betroffen war. Auch histologische Untersuchungen bestätigten die makroskopischen Erhebungen. Das günstigste histopathologische Bild konnte für die Testgruppen A und G gefunden werden. Histologisch zeigten die Testgruppen A und G als einzige von allen Gruppen nach 4 Wochen keine Restnekrosen mehr. Die zusammenfassenden Befunde der Gruppen A und G ergaben folgendes Bild:
- die Läsionsgrösse war im Vergleich zu den anderen Testgruppen nach 24 Stunden unverändert;
- die Breite des Ödems war hingegen deutlich reduziert, es erfolgte eine beschleunigte Rückbildung;
- es gab keine Restnekrosen im Corium;
- Testgruppe G zeigte ausserdem eine glattere und elastischere Gewebeneubildung im Vergleich zu allen übrigen Testgruppen.

In einem Beobachtungszeitraum von 24 Stunden konnte eindeutig der positive Einfluss der in Liposomen inkorporierten rhSOD gezeigt werden, obwohl die Dosis der rhSOD in den Testgruppen A und G zehnmal geringer war als bei den Testgruppen B und C. Die Nachbrennerscheinungen der Verbrühungswunden sowie insbesondere auch das Ödem, bildeten sich am schnellsten und deutlichsten in den mit liposomal inkorporierter rhSOD behandelten Gruppen zurück.

### Beispiel 2: Anwendung bei Verbrennungen

Ein Versuch mit Setzen von Verbrennungswunden wurde analog zur Methode von M. Choi und H.P. Ehrlich (American Journal of Pathology 142, 1993, 519-529) durchgeführt. 40 Ratten (Wistar, männliche Ratten) zu 300-350 g Körpergewicht wurden unter Standardbedingungen gehalten. Die anästhesierten Ratten (Anästhetikum: Pentobarbital, i.p.) wurden mit einem Stempel, der in kochendem Wasser erhitzt wurde, Brandwunden in Form eines Streifenmusters gesetzt. Zu diesem Zweck wurde der Stempel 30 sec. auf die rasierte Haut der Tiere gedrückt. Um das gewünschte Muster auf der Haut zu erhalten war der Stempel nach Choi und Ehrlich (1993) kammartig ausgebildet.

Die Ratten wurden in 4 Gruppen zu je 10 Tieren eingeteilt (Tabelle 2).

**Tabelle 2**

| Testgruppe | Anzahl der Tiere | Verabreichung |
|---|---|---|
| 1 | 10 | Kontrollgruppe: ohne Behandlung |
| 2 | 10 | Trägergel mit leeren Liposomen |
| 3 | 10 | Gel mit rhSOD in Liposomen |
| 4 | 10 | rhSOD in Liposomen mit Hyaluronsäure im Trägergel |

Die Herstellung der Liposomen und der Gele erfolgte analog zu Beispiel 1. Die Ratten wurden innerhalb von 48 Stunden sechsmal in regelmässigen Abständen behandelt, wobei die erste Behandlung wenige Minuten nach der Läsionssetzung erfolgte. Liposomal verpackte rhSOD wurde in einer Menge von 0.05 mg/cm² der Läsion eingesetzt, der Anteil an Hyaluronsäure in der verwendeten Zubereitung betrug 3 Gew.%:
Als Beurteilungskriterium für die potentielle Wirkung wurden die Planimetrie und die Makromorphologie des Wundmusters, das durch das Design des Stempels hervorgerufen wird, herangezogen. Weiters wurde eine Standardhistologie (Hämatoxylin-, Eosin- und Vitalfärbung; nach H.Millesi, 1970, Chirurgia Plastica et Reconstructia Vol. 8, Springer Verlag Berlin, Heidelberg, New York) der Gewebsproben durchgeführt. Es wurden nach 0 h, 25 h, 72 h, 7 Tagen und 21 Tagen Proben genommen.

Bei den Gruppen 3 und 4 blieb das durch die Stempelform bedingte Streifenmuster erhalten, d.h. dass die verbrannten Streifen vital erhalten bleiben. Bei den Gruppen 1 und 2 hingegen zeichnete sich eine Konfluenz zu einer mehr oder weniger einheitlichen Wundfläche ab. Bei den Gruppen 3 und 4 zeichnete sich auch ein günstiges histopathologisches Bild ab. Bei den mit in Liposomen inkorporierter rhSOD behandelten Gruppen blieb das Muster der nicht exponierten Streifen erhalten. Die vom Stempeldruck betroffenen Flächen wurden durch Granulations- und Narbengewebe ersetzt, das mehrheitlich nekrosefrei war. Bei den Gruppen 1 und 2 war eine einheitliche nicht mehr gemusterte Narbenfläche, mit teilweise tiefen Nekrosen, zu beobachten.

### Beispiel 3: Anwendung bei Strahlenschäden

Haarlose Mäuse wurden einer UV-Strahlung gemäss der minimalen Erythemdosis (MED) ausgesetzt. Der Versuchsaufbau wurde wie bei B.C. Pence and M.F. Naylor (B.C. Pence, M.F. Naylor 1993, J.of Investigative Dermatology 95: 213-16) beschrieben, durchgeführt. Eine Einzeldosis von UVB- Strahlung (290-320 nm) einer Westinghouse FS-40 Sonnenlampe wurde so eingesetzt, dass die Mäuse einer totalen Energie von 0.09 J/cm² ausgesetzt waren. Diese Dosis entspricht der dreifachen MED für kaukasische Probanden. Es wurden 40 Mäuse in 4 Gruppen zu je 10 Tieren eingesetzt (Tab 3).

**Tabelle 3**

| Testgruppe | Anzahl der Tiere | Verabreichung |
|---|---|---|
| 1 | 10 | Kontrollgruppe: ohne Behandlung |
| 2 | 10 | Trägergel mit leeren Liposomen |
| 3 | 10 | Gel mit rhSOD in Liposomen |
| 4 | 10 | rhSOD in Liposomen mit Hyaluronsäure im Trägergel |

Die Liposomen und Gele wurden analog zu Beispiel 1 hergestellt. Das Gel wurde unmittelbar (2-3 min.) nach der Strahlenexposition und dann noch zweimal in regelmässigen Abständen von 4 Stunden aufgetragen. Das Gel in der verwendeten Zubereitung enthielt 0.5 Gew.% Hyaluronsäure, die verabreichte Dosis an rhSOD betrug 0.5 mg/cm² bestrahlter Körperoberfläche. Als Beurteilungskriterium wurde ein visueller Farbvergleich aller Tiere in regelmässigen Zeitabständen durchgeführt. Weiters wurde das Auftreten von "sunburn-cells' getestet.

Bei den Gruppen 3 und 4 trat nach 10 Stunden ein im Vergleich zu den Gruppen 1 und 2 signifikant geringeres Erythem auf. Nach 24 Stunden wurden Gewebeproben entnommen und auf die charakteristischen "sunburn-cells" hin untersucht. Bei den Gruppen 3 und 4 traten keine oder nur vereinzelte "sunburn-cells" auf. Die Rötungsintensität konnte mit dem Auftreten von sunburn-cells korrelativ in Beziehung gesetzt werden.

### Beispiel 4: Vergleich verschiedener Applikationsformen

140 männliche OF1 (outbred) Mäuse (vom Institut für Versuchstierzucht und - haltung, Himberg, Österreich), alle zwischen 8 - 10 Wochen alt, wurden nach dem Zufallsprinzip in 14 Gruppen (A-N) eingeteilt. Die Mäuse wurden unter Ethernarkose mit 50 µl PBS-Lösung, welche 1x10⁵ PFU/Maus (= 2-2.5 x LD₅₀) des Influenzavirus A/WSN/33 (nachfolgend als WSN bezeichnet) enthielt, intranasal inokuliert. Die Behandlung der WSN-infizierten Mäuse begann am Tag 4 nach der Infektion, d.h. am Beginn des Auftretens klinischer Symptome der Influenza-Erkrankung der Mäuse. Die Mäuse wurden 1x täglich bis zum Tag 11 nach der Infektion behandelt. Sie wurden topisch, subkutan, intravenös und intranasal mit Präparaten behandelt, welche rhSOD in folgenden liposomalen Vesikelarten beinhalteten:
a) rhSOD in kleinen, unilamellaren Vesikeln (SUV), Grösse ≤ ca. 100 nm,
b) rhSOD in Injektionsvesikeln (IV), Durchmesser ≤ ca. 200 nm, und
c) rhSOD in multilamellaren Vesikeln (MLV), Durchmesser ≤ ca. 500 nm.

Tabelle 4 zeigt die Versuchsanordnung und die erzielten Ergebnisse. Zur topischen Behandlung wurde ein liposomales rhSOD-Gel auf der ventralen Seite der Mäuse, speziell im Brustbereich, durch Einschmieren 1x pro Tag aufgetragen. Es wurde dadurch ein Hautbereich von ca. 10 cm² mit einer ungefähr 2 mm dicken Gel-Schicht bedeckt. Die Konzentrationsangaben in Tabelle 4 beziehen sich dabei auf die behandelte Fläche in cm². Die intranasale Applikation von rhSOD fand unter Ethernarkose der Tiere statt. Es wurden dabei 0.05 ml einer Suspension mit rhSOD in IV (1 mg/ml) mittels einer Mikropipette in the Nasenlöcher der Mäuse eingebracht.
Zur intravenösen Verabreichung der rhSOD-Suspensionen (siehe Tabelle 4) wurde in die Schwanzvene injiziert, während zur subkutanen Applikation dieselben Suspensionen wie bei der i.v. Injektion in den Hals- und/oder Schulterbereich subkutan injiziert wurden.
Die Anzahl der Todesfälle der Mäuse erhöhte sich nach dem 15. Tag seit der Infektion nicht mehr weiter (getesteter Zeitraum: Tag 15 bis Tag 25 nach der Infektion), sodass Mäuse, welche am 15. Tag überlebt haben, als von der Influenza-Infektion geheilt angesehen wurden.

Die Pathogenese einer Influenzavirus-Infektion bei Mäusen zeigt eher eine Überreaktion der Immunabwehr des Wirtsorganismus als einen direkten Effekt bezüglich der Virusvermehrung. Die Entstehung freier Sauerstoffradikale im Aktivität in den Lungen und im Serum der Mäuse beeinflusst (Oda et al., Science. 1989. 244(4907):974-6).

| Grupp e | Applikationsform und Dosis | Überlebend e je 10 Mäuse |
|---|---|---|
| A | topische Applikation eines Gels von rhSOD inkorporiert in SUV; D'osis: 0,15 mg rhSOD/cm² Behandlungsfläche | 4 von 10 |
| B | topische Applikation eines Gels von rhSOD inkorporiert in IV; Dosis: 0,15 mg rhSOD/cm² Behandlungsfläche | 4 von 10 |
| C | topische Applikation eines Gels von rhSOD inkorporiert in MLV; Dosis: 0,42 mg rhSOD/cm² Behandlungsfläche | 3 von 10 |
| D | topische Applikation eines Gels mit IV ohne rhSOD | 2 von 10 |
| E | s.c. Applikation einer Suspension von rhSOD inkorporiert in SUV | 8 von 10 |
| | Dosis:of 0,5 ml; Suspension: 1 mg rhSOD/ml | |
| F | s.c. Applikation einer Suspension von rhSOD inkorporiert in IV | 8 von 10 |
| | Dosis:of 0,5 ml; Suspension: 1 mg rhSOD/ml | |
| G | s.c. Applikation einer Suspension von rhSOD inkorporiert in MLV | 5 von 10 |
| | Dosis:of 0,5 ml; Suspension: 3 mg rhSOD/ml | |
| H | s.c. Applikation von 0,5 ml einer IV-Suspension ohne rhSOD | 1 von 10 |
| I | i.v. Applikation einer Suspension von rhSOD inkorporiert in SUV | 9 von 10 |
| | Dosis:of 0,5 ml; Suspension: 1 mg rhSOD/ml | |
| J | i.v. Applikation einer Suspension von rhSOD inkorporiert in IV | 10 von 10 |
| | Dosis:of 0,5 ml; Suspension: 1 mg rhSOD/ml | |
| K | i.v. Applikation einer Suspension von rhSOD inkorporiert in MLV | 4 von 10 |
| | Dosis:of 0,5 ml; Suspension: 3 mg rhSOD/ml | |
| L | i.v. Applikation von 0,5 ml einer IV-Suspension ohne rhSOD | 1 von 10 |
| M | i.n. Applikation einer Suspension von rhSOD inkorporiert in IV | 8 von 10 |
| | Dosis:of 0,05 ml; Suspension: 1 mg rhSOD/ml | |
| N | Kontrollgruppe ohne Behandlung | 1 von 10 |

### Resultat:

Mäuse, die parenteral (s.c., i.v. und i.n.) mit rhSOD in SUV oder IV-Liposomen behandelt wurden, überlebten in den meisten Fällen (Gruppen E, F, I, J, M) im Vergleich mit den Kontrollgruppen, welche entweder überhaupt nicht oder nur mit Liposomen ohne SOD behandelt wurden (Gruppen H, L, und N). Es zeigte sich überraschenderweise ausserdem, dass Mäuse, die mit rhSOD in MLV behandelt wurden, weniger gut gegen die tödliche Influenza-lnfektion geschützt waren, obwohl in diesen Liposomenpräparationen eine höhere rhSOD-Menge eingesetzt wurde (Gruppen C, G, und K). Der Grund dafür liegt wahrscheinlich in der Vesikelgrösse (im Bereich von 500 nm; zum Vergleich: IV ≤ ca. 200 nm; SUV ≤ ca. 100 nm), schlechterer Verteilung und geringerer Fusionsfähigkeit der MLV mit den Zellen, wie anhand eines *in vitro*-Fusionsassays festgestellt werden konnte (Fussionsassay: CHO-Zellen wurden über Nacht mit 20 µmol rhSOD-hältigen IV und MLV-Liposomen inkubiert, rhSOD wurde durch Immunfluoreszenz nach Behandlung mit 1% Triton X100 detektiert).

Die topische Behandlung der Influenza-infizierten Mäuse mit rhSOD-Gel erzeugte nur einen teilweisen Schutz gegen die tödlichen Folgen der Erkrankung; dennoch war dieser Schutz signifikant in den Gruppen, die mit SUV oder IV-Präparationen (Gruppen A und B) behandelt wurden. Die Resultate zeigen, dass freie Sauerstoffradikale eine bedeutende Rolle in Hinblick auf die tödlichen Auswirkungen der Influenzainfektion bei Mäusen spielen und dass rhSOD, inkorporiert in kleine liposomale Vesikel (SUV und IV), ein beachtliches therapeutisches Potential gegenüber dieser Virusinfektion besitzt.

Die Resultate zeigen ausserdem, dass rhSOD, inkorporiert in kleine Liposomen (SUV, IV) mit einem Durchmesser von rund 200 nm und darunter, offensichtlich die Hautbarrieren selbst bei im wesentlichen gesunder, intakter Haut überwinden und in tiefere Gewebeschichten penetrieren kann, um so seine schützende Wirkung gegenüber der Virusinfektion im Lungenbereich (Lungenentzündung) zu entfalten. Liposomal inkorporierte rhSOD kann daher nicht nur für i.v., s.c. und i.n. Applikationen, sondern auch in Form von Salben, Gelen, Crèmes oder anderen geeigneten Formulierungen, gegebenenfalls in Kombination mit anderen Wirkstoffen und/oder inklusive weiterer Zusatzstoffe, als ein wirksames Therapeutikum zur Behandlung von Krankheiten eingesetzt werden, die im Zusammenhang mit freien Sauerstoffradikalen stehen, beispielsweise von - gegebenenfalls mikrobiell verursachten - Entzündungen, insbesondere der oberen Atemwege und der Lunge.

### Beispiel 5: Topische Anwendung bei Herpes labialis

In einem Humanversuch wurde rhSOD, inkorporiert in kleine, unilamellare Vesikeln ("small unilamellar vesicles", SUV) mit einem Vesikeldurchmesser von rund 100 nm oder darunter, gegen eine lokale Hautentzündung im Mundbereich, verursacht durch Fieberbläschen auf der Lippe (herpes labialis), getestet. Herpesinfektionen und Herpeserkrankungen sind bekanntermassen schwierig zu behandeln und für die Betroffenen zumeist sehr unangenehm, da sie häufig Schmerzen (z.B. bei herpes zoster), Jucken, Brennen, Nässeln und/oder kosmetisch unvorteilhafte Auswirkungen, speziell im Gesichtsbereich, verursachen.

Die liposomale rhSOD-Präparation auf Basis des Carbopol® -Gels hatte eine hautcremeartige Konsistenz und wurde in einer Konzentration von rund 0.15 mg rhSOD pro cm² Behandlungsfläche ein- bis zweimal pro Tag direkt auf den entzündeten Bereich aufgetragen. Bereits nach der ersten Anwendung zeigte sich innerhalb weniger Stunden eine spührbare Erleichterung und schon nach 3-tägiger Behandlung waren die typischen Symptome weitestgehend abgeklungen.

### Beispiel 6: Topische Anwendung gegen Allergie

Dieselbe rhSOD-Präparation wie in Beispiel 5 wurde in einem anderen Humanversuch gegen allergische Reaktionen der Haut getestet. Die Allergiesymptome waren gerötete Augen und Schwellungen rund um die Augen und im Bereich zwischen Augen und Nase, möglicherweise als zusätzliche Auswirkungen einer bestehenden Heuschnupfenallergie. Neben den durch die Schwellungen verursachten unangenehmen Spannungen im Gesicht und dem zusätzlich damit verbundenen psychologischen Leidensdruck war ausserdem eine Beeinträchtigung des normalen Sehens gegeben.

Das rhSOD-Gel wurde, analog zu Beispiel 5, in einer Konzentration von ca. 0.15 mg rhSOD pro cm² Behandlungsfläche im gesamten betroffenen Gesichtsbereich wie eine Hautcreme aufgetragen. Das Ergebnis war beeindruckend: Bereits nach einmaligem Auftragen gingen die Schwellungen fast vollständig zurück; sicherheitshalber wurde am darauf folgenden Tag zwar noch ein zweites Mal eingecremt, eine weitere Behandlung war jedoch nicht mehr nötig. Die Beschwerden sind praktisch vollständig abgeklungen.

### Beispiel 7: Anwendung gegen Psoriasis

In einem weiteren Versuch wurde das rhSOD-Gel aus Beispiel 5 an einem kindlichen Patienten gegen Psoriasis eingesetzt. Das Präparat wurde, analog zu Beispiel 5, in einer Konzentration von ca. 0.15 mg rhSOD pro cm² Behandlungsfläche an allen betroffenen Körperstellen 2x täglich (am Morgen und am Abend) wie eine Hautcreme aufgetragen.

Resultat: Nach drei Tagen Behandlung zeigte sich ein deutlicher Rückgang der Hautrötungen im Bereich der Entzündungsherde sowie eine Verminderung des Juckreizes. Dieser erfreuliche Befund zeigt, dass rhSOD in Liposomen bei externer topischer Applikation auch bei Psoriais-Patienten - wenn schon nicht zur Heilung der Krankheit - so doch zumindest zur Linderung der Symptome erfolgreich eingesetzt werden kann.

## Patentansprüche

1. Verwendung von rekombinanter humaner Cu,Zn-SOD (rhSOD) in Liposomen, in Mischung mit Hyaluronsäure, für die Herstellung einer pharmazeutischen Zubereitung zur prophylaktischen und/oder therapeutischen Anwendung gegen erhöhte Konzentrationen an Superoxidradikalen und/oder dadurch verursachte Schädigungen am menschlichen Körper und Teilen davon.

2. Verwendung von rekombinanter humaner Cu,Zn-SOD (rhSOD) in Liposomen für die Herstellung einer pharmazeutischen Zubereitung zur prophylaktischen und/oder therapeutischen Anwendung gegen erhöhte Konzentrationen an Superoxidradikalen und/oder dadurch verursachte Schädigungen am menschlichen Körper und Teilen davon.

3. Verwendung nach Anspruch 1 oder 2, für die Herstellung einer pharmazeutischen Zubereitung zur - insbesondere äusserlichen - Anwendung gegen Strahlenschädigungen der Haut und benachbarter Gewebe, bei Verbrennungen, Verbrühungen, entzündlichen Prozessen, insbesondere entzündlichen Hauterkrankungen, sowie rheumatischarthritischen Erkrankungen.

4. Verwendung nach Anspruch 1 oder 2 für die Herstellung einer pharmazeutischen Zubereitung zur Anwendung gegen rheumatische Gelenksentzündungen und/oder Osteoarthritis.

5. Verwendung nach einem der Ansprüche 1 oder 2 für die Herstellung einer pharmazeutischen Zubereitung zur Anwendung gegen Hautemphyseme, Furunkeln, Akne oder mikrobiell, insbesondere durch Viren, verursachte Entzündungen.

6. Verwendung nach Anspruch 5 zur Herstellung einer pharmazeutischen Zubereitung zur Anwendung gegen Entzündungen, die durch Influenzavirus verursacht werden, insbesondere im Bereich der Atemwege und der Lunge, oder durch Herpesviren, insbesondere im Bereich von Mund und Lippen (herpes labialis).

7. Verwendung nach Anspruch 1 oder 2 für die Herstellung einer pharmazeutischen Zubereitung zur Anwendung, vorzugsweise per Inhalation, gegen Entzündungen der Atemwege und der Lunge, insbesondere gegen Bronchitis, Schocklunge (ARDS) und/oder Lungenemphysem.

8. Verwendung nach Anspruch 1 oder 2 zur Herstellung einer pharmazeutischen Zubereitung zur vorzugsweise topischen Anwendung gegen - insbesondere allergisch bedingte - Rötungen und Schwellungen der Haut oder gegen Psoriasis.

9. Verwendung nach einem der Ansprüche 1 bis 3, worin die Zubereitung ausserdem mindestens ein strahlenfilterndes oder strahlenabsorbierendes Schutzmittel, vorzugsweise ein Lichtfilter oder UV-Absorber, insbesondere ein UVB-Filter, sowie gegebenenfalls weitere, vorzugsweise hautpflegende Faktoren, enthält.

10. Verwendung nach einem der vorhergehenden Ansprüche, **dadurch gekennzeichnet, dass** die pharmazeutische Zubereitung in Form von Emulsionen, Suspensionen, Lösungen, Lotions, Salben, Gelen oder durch Aufsprühen aus einer Sprühflasche oder einer sonstigen Sprühvorrichtung appliziert wird.

11. Verwendung von rekombinanter humaner Cu,Zn-SOD (rhSOD) in Liposomen, in Mischung mit Hyaluronsäure, für die Herstellung einer kosmetischen Zubereitung zur äusseren Anwendung, insbesondere als Hautpflege- und/oder Sonnenschutzmittel.

12. Verwendung von rekombinanter humaner Cu,Zn-SOD (rhSOD) in Liposomen, gegebenenfalls in Mischung mit Hyaluronsäure, für die Herstellung einer Zubereitung zur Verbesserung der Haltbarkeit von organischen Materialien.

13. Verwendung nach einem der Ansprüche 1, 2 oder 12 für die Herstellung einer Zubereitung zur Anwendung bei organischen Geweben, Organen, und Organ- bzw. Gewebetransplantaten.

14. Verwendung nach Anspruch 12 für die Herstellung einer Zubereitung zur Anwendung bei Lebensmitteln, vorzugsweise leichtverderblichen Fleisch- und Molkereiprodukten, oder bei kosmetischen Präparaten auf organischer Basis, insbesondere bei Hautpflegemitteln in Form von Salben, Crémes, Gelen, Wässern, Ölen oder Lotionen.

15. Verwendung nach einem der vorhergehenden Ansprüche, worin die rhSOD für äusserliche Anwendungen in einer Dosis von 0.01-1 mg/cm² behandelter Körperoberfläche, für andere, insbesondere orale oder parenterale, Applikationen in einer Dosis von 0.5-50 mg/kg Körpergewicht eingesetzt wird.

16. Verwendung nach einem der vorhergehenden Ansprüche, worin in der Zubereitung ausserdem ein fettarmer oder fettfreier Träger, vorzugsweise aus der Gruppe der organischen und anorganischen Hydrogele, enthalten ist.

17. Verwendung nach einem der vorhergehenden Ansprüche, worin die rhSOD in einer Konzentration von grösser oder gleich 0.01 Gew.%, insbesondere von 0.01 bis 5 Gew.%, der Zubereitung vorliegt.

18. Verwendung nach einem der Ansprüche 1, 3 bis 13 worin Hyaluronsäure in einer Konzentration von grösser oder gleich 0.05 Gew.%, insbesondere von 0.1 bis 5 Gew.%, der Zubereitung vorliegt.

19. Verwendung nach einem der vorhergehenden Ansprüche mit rhSOD in Liposomen, wobei die Liposomen eine durchschnittliche Grösse von weniger als 600 nm, vorzugsweise weniger als 300 nm, insbesondere weniger als 150 nm, aufweisen und vorzugsweise unilamellar sind.

20. Verfahren zur Verbesserung der Haltbarkeit von organischen Materialien, **dadurch gekennzeichnet, dass** eine Zubereitung, welche rekombinante humane Cu,Zn-SOD (rhSOD), gegebenenfalls in Liposomen und/oder in Mischung mit Hyaluronsäure enthält, mit dem organischen Material zumindest teilweise in Kontakt gebracht wird.

21. Verfahren nach Anspruch 20 worin die Zubereitung flüssig ist und mittels einer Sprühvorrichtung auf das organische Material aufgesprüht oder das organische Material in die flüssige Zubereitung eingetaucht oder eingelegt wird, oder im Falle von Organen und Organtransplantaten, diese nach üblichen Methoden der Organkonservierung, insbesondere durch Injektion oder Infusion, gespült werden.

22. Verfahren nach einem der Ansprüche 20 oder 21, worin das organische Material tierische oder pflanzliche Gewebe, vorzugsweise Organe, Organtransplantate, Gewebetransplantate und/oder Lebensmittel, insbesondere leichtverderbliche Fleisch- und Molkereiprodukte, umfasst.

23. Verfahren nach Anspruch 20 oder 21, worin die Zubereitung zu organischem Material in einem kosmetischen Präparat hinzugefügt wird.

24. Verfahren nach einem der Ansprüche 20 bis 23, worin die rhSOD in einer Konzentration von grösser oder gleich 0.01 Gew.%, insbesondere von 0.01 bis 5 Gew.%, und gegebenenfalls Hyaluronsäure in einer Konzentration von grösser oder gleich 0.05 Gew.%, insbesondere von 0.1 bis 5 Gew.%, der Zubereitung vorliegt.

25. Verfahren nach einem der Ansprüche 20 bis 24, **dadurch gekennzeichnet, dass** die rhSOD in einer Menge von 0.1 - 100 mg/kg organisches Material angewendet wird.

26. Kosmetische oder pharmazeutische Zusammensetzung enthaltend rekombinante humane Cu,Zn-SOD in Liposomen in Mischung mit Hyaluronsäure.

## Claims

1. Use of recombinant human Cu,Zn-SOD (rhSOD) in liposomes, as a mixture with hyaluronic acid, for the preparation of a pharmaceutical formulation for prophylactic and/or therapeutic application to treat elevated concentrations of superoxide radicals and/or damage caused thereby in the human body and parts thereof.

2. Use of recombinant human Cu,Zn-SOD (rhSOD) in liposomes for the preparation of a pharmaceutical formulation for prophylactic and/or therapeutic application to treat elevated concentrations of superoxide radicals and/or damage caused thereby in the human body and parts thereof.

3. Use according to Claim 1 or 2, for the preparation of a pharmaceutical formulation for - in particular external - application to treat radiation damage to the skin and adjacent tissue, in the case of burns, scalds, inflammatory processes, in particular inflammatory skin diseases, and for rheumatic-arthritic diseases.

4. Use according to Claim 1 or 2, for the preparation of a pharmaceutical formulation for application to treat rheumatic inflammation of joints and/or osteoarthritis.

5. Use according to either of Claims 1 and 2, for the preparation of a pharmaceutical formulation for application to treat subcutaneous emphysema, furuncles, acne or microbially caused inflammations, in particular inflammations caused by viruses.

6. Use according to Claim 5, for the preparation of a pharmaceutical formulation for application to treat inflammations caused by an influenza virus, in particular in the region of the respiratory tract and the lung, or by herpes viruses, in particular in the region of the mouth and lips (herpes labialis).

7. Use according to Claim 1 or 2, for the preparation of a pharmaceutical formulation for application, preferably by inhalation, to treat inflammations of the respiratory tract and of the lung, in particular to treat bronchitis, acute respiratory distress syndrome (ARDS) and/or pulmonary emphysema.

8. Use according to Claim 1 or 2, for the preparation of a pharmaceutical formulation for preferably topical application to treat - in particular allergy-related - redness and swelling of the skin or to treat psoriasis.

9. Use according to any of Claims 1 to 3, wherein the formulation additionally contains at least one radiation-filtering or radiation-absorbing protective agent, preferably a light filter or UV absorber, in particular a UVB filter, and optionally further, preferably skin-care factors.

10. Use according to any of the preceding Claims, **characterized in that** the pharmaceutical formulation is applied in the form of emulsions, suspensions, solutions, ointments or gels or by spraying on from a spray bottle or another spray apparatus.

11. Use of recombinant human Cu,Zn-SOD (rhSOD) in liposomes, as a mixture with hyaluronic acid, for the preparation of a cosmetic formulation, for external application, in particular as a skin-care agent and/or a sunscreen agent.

12. Use of recombinant human Cu,Zn-SOD (rhSOD) in liposomes, optionally as a mixture with hyaluronic acid, for the preparation of a formulation for improving the stability of organic materials.

13. Use according to any of Claims 1, 2 and 12, for the preparation of a formulation for application in the case of organic tissues, organs and organ or tissue transplants.

14. Use according to Claim 12, for the preparation of a formulation for application in the case of foods, preferably easily perishable meat and dairy products, or in the case of cosmetic preparations based on organic material, in particular in the case of skin-care agents in the form of ointments, creams, gels, waters, oils or lotions.

15. Use according to any of the preceding Claims, wherein the rhSOD is used in a dose of 0.01-1 mg/cm² of treated body surface for external applications and in a dose of 0.5-50 mg/kg body weight for other, in particular oral or parenteral, applications.

16. Use according to any of the preceding Claims, wherein a low-fat or fat-free excipient, preferably from the group consisting of the organic and inorganic hydrogels, is additionally contained in the formulation.

17. Use according to any of the preceding Claims, wherein the rhSOD is present in a concentration greater than or equal to 0.01% by weight, in particular of 0.05 to 5% by weight, of the formulation.

18. Use according to any of Claims 1 and 3 to 13, wherein hyaluronic acid is present in a concentration greater than or equal to 0.05% by weight, in particular of 0.1 to 5% by weight, of the formulation.

19. Use according to any of the preceding Claims, comprising rhSOD in liposomes, the liposomes having an average size of less than 600 nm, preferably less than 300 nm, in particular less than 150 nm, and preferably being unilaminar.

20. Method for improving the stability of organic materials, **characterized in that** a formulation which contains recombinant human Cu,Zn-SOD (rhSOD), optionally in liposomes and/or as a mixture with hyaluronic acid, is brought at least partly into contact with the organic material.

21. Method according to Claim 20, wherein the formulation is liquid and is sprayed onto the organic material by means of a spray apparatus or the organic material is dipped into or placed in the liquid formulation or, in the case of organs and organ transplants, said organs and organ transplants are irrigated by customary methods of organ preservation, in particular by injection or infusion.

22. Method according to either of Claims 20 and 21, wherein the organic material comprises animal or vegetable tissues, preferably organs, organ transplants, tissue transplants and/or food, in particular easily perishable meat and dairy products.

23. Method according to Claim 20 or 21, wherein the formulation is added to organic material in a cosmetic preparation.

24. Method according to any of Claims 20 to 23, wherein the rhSOD is present in a concentration greater than or equal to 0.01% by weight, in particular of 0.01 to 5% by weight, and optionally hyaluronic acid is present in a concentration greater than or equal to 0.05% by weight, in particular of 0.1 to 5% by weight, of the formulation.

25. Method according to any of Claims 20 to 24,
**characterized in that** the rhSOD is applied in an amount of 0.1-100 mg/kg of organic material.

26. Cosmetic or pharmaceutical composition, containing recombinant human Cu,Zn-SOD in liposomes as a mixture with hyaluronic acid.

## Revendications

1. Utilisation d'une Cu,Zn-SOD humaine de recombinaison (SOD-hr) dans des liposomes, en mélange avec de l'acide hyaluronique pour la réalisation d'une préparation pharmaceutique à usage prophylactique et / ou thérapeutique contre les concentrations élevées de radicaux superoxyde et / ou les dommages causés par ces radicaux sur le corps humain ou des parties du corps humain.

2. Utilisation d'une Cu,Zn-SOD humaine de recombinaison (SOD-hr) dans des liposomes pour la réalisation d'une préparation pharmaceutique à usage prophylactique et / ou thérapeutique contre les concentrations élevées de radicaux superoxyde et / ou les dommages causés par ces radicaux sur le corps humain ou des parties du corps humain.

3. Utilisation selon la revendication 1 ou la revendication 2, pour la réalisation d'une préparation pharmaceutique - en particulier à usage externe - active contre les atteintes de la peau et des tissus adjoignants consécutives à des radiations, des brûlures, des brûlures par liquides, des processus inflammatoires, en particulier des maladies inflammatoires de la peau, ainsi que des maladies du type rhumatismes / arthrites.

4. Utilisation selon la revendication 1 ou la revendication 2, pour la réalisation d'une préparation pharmaceutique active contre les rhumatismes / inflammations articulaires et / ou les arthroses.

5. Utilisation selon la revendication 1 ou la revendication 2, pour la réalisation d'une préparation pharmaceutique active contre les emphysèmes cutanés, les furoncles, l'acné, ou les inflammations causées par des microbes et surtout par des virus.

6. Utilisation selon la revendication 5, pour la réalisation d'une préparation pharmaceutique active contre les inflammations provoquées par le virus de la grippe, en particulier au niveau des voies respiratoires et des poumons ou par le virus de l'herpès, en particulier au voisinage de la bouche et des lèvres (herpes labialis).

7. Utilisation selon la revendication 1 ou la revendication 2, pour la réalisation d'une préparation pharmaceutique pour une administration, de préférence par inhalation, contre les inflammations des voies respiratoires et des poumons, en particulier contre la bronchite, le syndrome de détresse respiratoire aiguë (ARDS), et / ou les emphysèmes pulmonaires.

8. Utilisation selon la revendication 1 ou la revendication 2, pour la réalisation d'une préparation pharmaceutique - de préférence à usage topique - active contre des rougeurs ou des enflures de la peau (en particulier d'origine allergique) ou contre le psoriasis.

9. Utilisation selon l'une des revendications 1 à 3, où la préparation contient, en outre, au moins un agent de protection antisolaire filtrant ou absorbant la lumière, de préférence un filtre pour la lumière ou un absorbant des UV, en particulier un filtre pour les UVB, ainsi que, le cas échéant d'autres agents, de préférence, des agents pour soins de la peau.

10. Utilisation selon l'une des revendications précédentes, **caractérisée en ce que** la préparation pharmaceutique est appliquée sous la forme d'une émulsion, d'une suspension, d'une solution, d'une lotion, d'un onguent, d'un gel ou d'un aérosol, d'une bombe aérosol ou d'un dispositif de pulvérisation approprié.

11. Utilisation d'une Cu,Zn-SOD humaine de recombinaison (SOD-hr) dans des liposomes, en mélange avec de l'acide hyaluronique, pour une préparation cosmétique à usage externe, en particulier une préparation pour le soin de la peau ou de protection solaire.

12. Utilisation d'une Cu,Zn-SOD humaine de recombinaison (SOD-hr) dans des liposomes, le cas échéant en mélange avec de l'acide hyaluronique, pour une préparation pour améliorer la conservation de matières organiques.

13. Utilisation selon l'une des revendications 1, 2 ou 12 pour réaliser une préparation destinée à des tissus organiques ou à des organes ou encore à des organes ou des tissus destinés à être transplantés.

14. Utilisation selon la revendication 12 pour la réalisation d'une préparation destinée à des produits alimentaires, de préférence des produits laitiers et carnés à courte conservation ou à des préparations cosmétiques à base organique, en particulier pour les soins de peau, sous la forme d'onguents, de crèmes, de gels, de préparations aqueuses, d'huiles ou de lotions.

15. Utilisation selon l'une des revendications précédentes, où la SOD-hr est appliquée topiquement à raison de 0,01 - 1 mg / cm² de surface de corps traitée ou par une autre voie, en particulier orale ou parentérale, à raison de 0,5 - 50 mg / kg de poids corporel.

16. Utilisation selon l'une des revendications précédentes, où la préparation contient, en outre, un excipient pauvre en matières grasses ou sans matières grasses choisi, de préférence, dans le groupe des hydrogels organiques et inorganiques.

17. Utilisation selon l'une des revendications précédentes, où la SOD-hr est présente dans la préparation à une concentration supérieure ou égale à 0,01 % en poids et, en particulier, allant de 0,01 à 5 % en poids.

18. Utilisation selon l'une des revendications 1, 3 à 13, où l'acide hyaluronique est présent dans la préparation à une concentration supérieure ou égale à 0,05 % en poids et, en particulier, allant de 0,1 à 5 % en poids .

19. Utilisation selon l'une des revendications précédentes de la SOD-hr dans des liposomes, où les liposomes ont une taille moyenne inférieure à 600 nm, de préférence inférieure à 300 nm et, surtout, inférieure à 150 nm et où les liposomes sont, de préférences, unilamellaires.

20. Procédé pour améliorer la conservation de matières organiques, **caractérisé en ce qu'**une préparation qui contient de la Cu,Zn-SOD humaine de recombinaison (SOD-hr), le cas échéant dans des liposomes et / ou en mélange avec de l'acide hyaluronique, est amenée, au moins partiellement, en contact avec ces matières organiques.

21. Procédé selon la revendication 20, dans lequel la préparation est liquide et est pulvérisée par un dispositif de pulvérisation sur les matières organiques, ou les matières organiques sont plongées ou maintenues dans la préparation liquide, ou encore, dans le cas d'organes et d'organes destinés à être transplantés, on utilise des méthodes de conservation habituelles pour les organes faisant appel à une perfusion ou à une injection.

22. Procédé selon la revendication 20 ou la revendication 21, où les matières organiques sont constituées par un tissu animal ou végétal et, de préférence, par un organe, un organe ou un tissu destiné à être transplanté et / ou un produit alimentaire, en particulier un produit carné ou laitier à courte conservation.

23. Procédé selon la revendication 20 ou la revendication 21, où la préparation est combinée avec des matières organiques dans une formulation cosmétique.

24. Procédé selon l'une des revendications 20 à 23, où la SOD-hr est présente en une concentration supérieure ou égale à 0,01 % en poids et, en particulier, allant de 0,01 à 5 % en poids et où l'acide hyaluronique est éventuellement présent à une concentration supérieure ou égale à 0,05 % en poids et, en particulier, allant de 0,1 à 5 % en poids de la préparation.

25. Procédé selon l'une des revendications 20 à 24, **caractérisé en ce que** la SOD-hr est utilisée à raison de 0,1 - 100 mg / kg de matières organiques.

26. Composition cosmétique ou pharmaceutique contenant de la Cu,Zn-SOD humaine de recombinaison dans des liposomes en mélange avec de l'acide hyaluronique.
